# EUROPEAN PATENT SPECIFICATION

(11) **EP 0 697 396 B1**
(45) Date of publication and mention of the grant of the patent: **14.04.1999**
(21) Application number: 95305279.2
(22) Date of filing: 28.07.1995
(51) Int. Cl.: C07C 209/84, C07C 209/86, B01D 15/08

(54) **Adsorption process for amine or amine salt recovery from aqueous brine solutions**
Verfahren zur Rückgewinnung von Aminen oder deren Salzen aus wässrigen Salzbrühen durch Adsorption
Procédé de récupération d'amines ou de leurs sels de saumures aqueuses par adsorption

(30) Priority: 17.08.1994 US 291635
(43) Date of publication of application: 21.02.1996
(73) Proprietor: GENERAL ELECTRIC COMPANY, Schenectady, NY 12345 (US)
(72) Inventor: Flowers, Larry Ivis, Evansville, Indiana 47711 (US); Boden, Eugene Pauling, Scotia, New York 12302 (US); Ramsey, David Lee, Mount Vernon, Indiana 47620 (US); Phelps, Peter David, Schenectady, New York 12306 (US)
(74) Representative: Szary, Anne Catherine, Dr.

(56) References cited:
- EP-A- 0 266 117
- GB-A- 1 472 966
- US-A- 4 913 812
- US-A- 5 071 560
- PATENT ABSTRACTS OF JAPAN vol. 017 no. 327 (C-1073) ,22 June 1993 & JP-A-05 032599 (SUMITOMO CHEM CO LTD) 9 February 1993,
- PATENT ABSTRACTS OF JAPAN vol. 017 no. 367 (C-1082) ,12 July 1993 & JP-A-05 058972 (SUMITOMO CHEM CO LTD) 9 March 1993,
- PATENT ABSTRACTS OF JAPAN vol. 017 no. 689 (C-1143) ,16 December 1993 & JP-A-05 229995 (SUMITOMO CHEM CO LTD) 7 September 1993,

## Description

The present invention relates to a process for the recovery of amines, amine salts, or mixtures thereof from aqueous brine solutions. More particularly the present invention relates to a process for the recovery of amines, amine salts, or mixtures thereof from by-product aqueous brine solutions resulting from the interfacial condensation polymerization of bisphenols and phosgene to form polycarbonates. In a preferred embodiment of the present invention, the instant process utilizes an adsorption resin whereby the amines, amine salts, or mixtures thereof present in an aqueous brine are adsorbed from the aqueous brine and then recovered by washing the adsorption resin having the amines, amine salts, or mixtures thereof adsorbed thereon with an aqueous solution having a lower ionic strength than the brine.

### Background of the Invention:

In the practice of interfacial polymerization to produce polycarbonates, a mixture of bisphenol and a phenolic chain-stopper is phosgenated under interfacial reaction conditions in the presence of an organic solvent, and in the presence of an effective amount of a catalyst which may be an organic amine or amine salt or mixtures thereof. The bisphenol is present as an alkali metal salt in an aqueous phase which is catalytically phosgenated by a suitable catalyst dissolved in an organic phase along with the phosgene. Suitable organic solvents which can be used are, for example, chlorinated aliphatic hydrocarbons, such as methylene chloride, chloroform, carbon tetrachloride, dichloroethane, trichloroethane, tetrachloroethane, dichloropropane and 1,2-dichloroethylene; substituted aromatic hydrocarbons such as, chlorobenzene, o-dichlorobenzene, and the various chlorotoluenes. The chlorinated aliphatic hydrocarbons, especially methylene chloride, are preferred

An effective amount of phase transfer catalyst is from 0.05 to 10.00 mole % based on the moles of bisphenol charged to the mixture. A preferred range of the phase transfer catalyst ranges from about 0.1 to about 0.7 mole % based on the total moles of bisphenol. When a co-catalyst is used, the quantity of co-catalyst ranges from about 0.001 to about 1.0 mole % based on the moles of bisphenol charged to the mixture.

Sufficient alkali metal hydroxide can be utilized to raise the pH of the bisphenol reaction mixture to 10.5 prior to phosgenation to provide dissolution of some of the bisphenol and chain-stopper into the aqueous phase. Aqueous alkali, or alkaline earth metal hydroxide can be used to maintain the pH of the phosgenation mixture which can be in the range of between about 7 to about 12.5 and preferably 10 to 12. Some of the alkali metal or alkaline earth metal hydroxides, which can be employed are for example, sodium hydroxide, potassium hydroxide, and calcium hydroxide. Sodium and potassium hydroxides and particularly sodium hydroxide is preferred.

Phosgenation of the bisphenol can be conducted in a wide variety of either batch or continuous reactors, Such reactors are, for example, stirred tank reactors, which may be either batch or continuous flow. Additional reactors which are included are agitated column and recirculating loop continuous reactors.

The volume ratio of aqueous to organic phase during and at the termination of the phosgenation reaction can be in the range of 0.2-1.1. Reaction temperature can be in the range of between about 15-50°C. When the preferred organic liquid is utilized, such as methylene chloride, the reaction may be conducted at reflux which can be 35°-42°C. The reaction can be conducted at atmospheric pressures, although sub- or super-atmospheric pressures may be employed if desired.

During phosgenation, the mixture is agitated, such as, by using a stirrer or other conventional equipment. The phosgenation rate can vary from between about 0.02-0.2 mole of phosgene, per mole of bisphenol per minute.

Depending upon the molecular weight of polycarbonate desired, phenolic chain-stoppers can be used in a proportion of from 1 to 8 mole % based on the total moles of bisphenol. Some of the phenolic chain-stoppers are, phenol, t-butyl phenol, p-cumylphenol and the chloroformates of these phenols.

Prior to polycarbonate recovery which can be achieved by standard techniques, such as filtration, decantation, and centrifugation, chloroformate groups are normally substantially eliminated. When a phase transfer catalyst is used by itself, the reaction mixture must be agitated for long periods of time until the presence of chloroformate groups can no longer be detected. Alternatively, the addition of an equivalent level of a phenolic compound based on the level of chloroformate can be added at the end of the reaction.

The aqueous phase contains alkali metal salts resulting from both the pH control of the reaction and the reaction products of the alkali metal salt of the bisphenol as well as water soluble chloroformate hydrolysis products. Thus, this aqueous phase is more properly characterized as a brine, being typically a brine having a fairly high ionic strength, usually anywhere from 10 to 30 weight percent sodium chloride. The amines, amine salts or mixtures thereof used as catalysts in the interfacial condensation polymerization may be soluble in both the aqueous and the organic phases. Thus while the organic phase may be conveniently recycled after recovery of the polycarbonate polymer, it may however be somewhat depleted in the concentration of the catalyst or catalyst mixture. That is, the catalysts used in the interfacial condensation polymerization of bisphenols and phosgene have a finite solubility in both liquid phases. Consequently as the process proceeds the amine or amine salt catalysts dissolve out of the organic phase and into the brine phase, frequently reaching concentrations in the brine phase as high as several parts per thousand. This results in an undesirable loss of an expensive catalytic component. Further, the presence of the amine or amine salt catalyst may render the brine unsuitable for many other uses even including simple dilution and disposal until the amine or amine salt has been extracted from the brine by some suitable process; heretofore typically either an energy intensive extractive steam distillation or a liquid-liquid extraction.

It is therefor desirable to provide a means of removing amines or amine salts or their mixtures from brine solutions. It is also desirable to be able to recover the amine or amine salt catalysts in a fashion that would permit their recycle and re-use as a catalyst.

### Summary of the Invention:

There is provided in the present invention a method for the recovery of amines or amine salt compounds from aqueous solutions having a high ionic strength by adsorbing the amine or amine salt onto an adsorption resin. The brine is thereby purified of the amines, amine salts or mixtures thereof. The adsorbed amines and/or amine salts are washed from the adsorption resin by means of another aqueous solution. More particularly there is provided in the present invention a process for the recovery and recycle of amines and/or amine salt phase transfer catalysts or mixtures thereof used in the interfacial condensation polymerization to produce polycarbonates.

The present invention provides a process for the adsorption of one or more amines and/or amine salt phase transfer catalysts from an aqueous solution having a high ionic strength comprising: (a) selecting an aqueous solution comprising:(i) a high ionic strength equivalent to a sodium chloride level present in said solution varying from about 5 weight percent to about 40 weight percent; and (ii) one or more amines and/ or amine salt compounds; (b) contacting said solution with a non-ion exchangeable adsorbent polymeric resin copolymer whereby said solution is depleted in the concentration of one or more of said amines and/or amine salt compounds by the adsorption of said amines and/or amine salt compounds onto said adsorbent polymeric resin copolymer.

The present invention also provides a process for the desorption of adsorbed amines and/or amine salt phase transfer catalysts, said one or more amines and/or amine salt compounds adsorbed onto an adsorbent polymeric resin copolymer comprising: (a) selecting a non ion exchangeable adsorbent polymeric resin copolymer having adsorbed thereon one or more amines and/or amine salts; and (b) desorbing said adsorbed amines, amine salts or mixtures thereof by washing said adsorbent polymeric resin copolymer with an effective amount of a solvent effective to remove said amines, amine salts, or mixtures thereof from said adsorbent polymeric resin copolymer, whereby said one or more amines, amine salts, or mixtures thereof are dissolved in said solvent.

The present invention also further provides a process for adsorption and desorption comprising : (a) adsorption of one or more amines and/or amine salt phase transfer catalysts from an aqueous solution having a high ionic strength comprising: (i) selecting an aqueous solution comprising: (1) a high ionic strength equivalent to a sodium chloride level present in said solution varying from about 5 weight percent to about 40 weight percent; and (2) said one or more amines, amine salts, or mixtures thereof; (ii) contacting said solution with an adsorbent polymeric resin copolymer whereby said solution is depleted in the concentration of said amines, amine salts, or mixtures thereof by the adsorption of said organic compounds onto said adsorbent polymeric resin copolymer; and (b) desorption of said adsorbed amines, amine salts, or mixtures thereof, said amines, amine salts or mixtures thereof adsorbed onto an adsorbent polymeric resin copolymer comprising: (i) selecting an adsorbent polymeric resin copolymer having adsorbed thereon one or more amines, amine salt compounds or mixtures thereof; and (ii) desorbing said adsorbed amines, amine salts, or mixtures thereof by washing said adsorbent polymeric resin copolymer with an effective amount of a solvent effective to remove said amines, amine salts, or mixtures thereof from said adsorbent polymeric resin copolymer, whereby said amines, amine salts, or mixtures thereof are dissolved in said solvent.

### Brief Description of the Drawing:

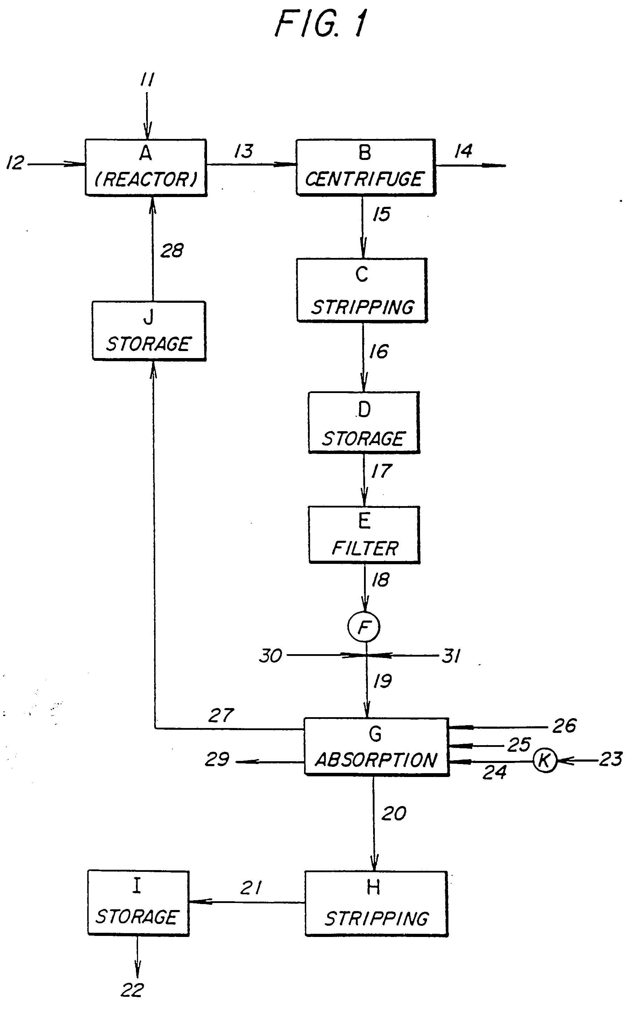

Figure 1 is a process flow diagram showing an interfacial process for the polymerization of bisphenol and phosgene derivatives to make polycarbonates along with the downstream product work-up units including the resin adsorption bed utilized in the process of the present invention. For purposes of clarity, the process units in the process flow scheme described by Figure 1 have been given alphabetic designations and the process streams have been given numerical designations.

### Detailed Description of the Invention:

The process of the present invention utilizes polymeric or co-polymeric resins and ion exchange resins that heretofore have been disclosed as suitable adsorbents for organic fluids as taught in U. S. patent 4,297,220 herein incorporated by reference. In contrast to the separation processes taught in U. S. patent 4,297,220 wherein organic fluids are separated one from the other by means of the ion exchange resins therein disclosed and claimed, the present invention provides for a process wherein amine or amine salts are selectively adsorbed from aqueous media as opposed to the non-aqueous media of the '220 patent on a resin that contains no ion exchange sites. More particularly, the present invention provides a process wherein the amine or amine salts or mixtures thereof are adsorbed from an aqueous medium having a high ionic strength and further said amines or amine salts or mixtures thereof are recovered by washing the resin with an aqueous solution having a low ionic strength. Additionally, the process of the present invention has a specific application in the interfacial condensation polymerization of bisphenol and phosgene derivatives to produce various polycarbonates wherein said amine or amine salts or mixtures thereof which function as phase transfer catalysts in said interfacial process are recycled and re-used by virtue of being recovered through the application of the process of the present invention. An environmental benefit ensues from the application of the present process, namely that the amine or amine salts present in the brine solution by-product resulting from the aforementioned interfacial polymerization are environmentally persistent and should be removed from the brine in some appropriate fashion before the brine may be diluted with water and discharged to the environment. Thus the present invention provides for two benefits, one being an improved efficiency in operating an interfacial polymerization process and the other being an environmental benefit in terms of reducing environmentally persistent effluents.

The process of the present invention is utilized for the adsorption, desorption and recovery of one or more of each of amines, amine salts or mixtures thereof. More preferably, the present invention is utilized for the adsorption, desorption, and recovery of one or more amines, present either singly or as a mixture. Most preferably, the present invention is utilized for the adsorption, desorption, and recovery of one or more amine salts, present either singly or as a mixture. Applicants note that the phrase amine salt refers to the ionic salts of primary, secondary, tertiary, and quaternary ammonium ions, and that these compounds are nitrogen compounds. The preferred amine salts recoverable by the process of the present invention are tertiary and quaternary ammonium salts. The bases for these varying preferences arise naturally from the selection of catalysts and catalysts employed in the interfacial polymerization of bisphenol and phosgene derivatives to produce the various polycarbonate polymers. Thus depending on the particular starting materials and end-product polymer desired, different catalyst systems will be employed depending on a variety of process and chemical factors such as kinetics, solubility, steric factors, degree of polymerization, and the like one catalyst system may be preferred over another.

The amine salts and tertiary amines useful either as catalysts or co-catalysts in the interfacial condensation polymerization of bisphenol and phosgene derivatives to produce polycarbonates present process management difficulties which the present invention alleviates. High molecular weight amine or amine salts have a tendency to function as surfactants in addition to fulfilling a catalytic role in the process, hindering the break-up of the polymerization emulsion, thus they generally are not preferred. When low molecular weight amine or amine salts are used as catalysts, the high solubility of the resulting organic ammonium salts render most materials ineffective for separating the ammonium compounds from the water solvent aggravating losses. By providing a means for recovering the amine or amine salts, the preferred lower molecular weight amine or amine salt phase transfer catalysts or co-catalysts may be more advantageously employed because losses due to aqueous solubility are minimized.

Some particular amine salts useful as phase transfer catalysts or co-catalysts are:

(R)₄N⁺X,

R¹(R)₃Q⁺X,

and

(R²)ₐ(R³)₃₋ₐN-(CH₂)ₙN-(R³)₃₋ₐ(R²)ₐ2X

where R is selected from the same or different C₍₃₋₁₀₎ alkyl group, R¹ is a C₍₁₋₃₎ alkyl group, R² is selected from the same or different C₍₁₋₂₎ alkyl group, R³ is selected from the same or different C₍₃₋₁₀₎ alkyl group, Q is a nitrogen or phosphorus atom, X can be a halogen atom, or an -OR⁴ group, R⁴ is a member selected from H, C₍₁₋₁₈₎ alkyl or C₍₆₋₁₈₎ aryl, and "a" is a whole number equal to 0 to 1, in addition to methyl tertiary amines and tri-amines such as tri-ethylamine which also function as catalysts. These organic amines, salts thereof and the like are amenable to recovery from brine solutions by the process of the present invention.

The adsorbents preferred for use in the process of the instant invention are suspension polymerization copolymers of monoethylenically unsaturated monomers and polyvinylidene monomers. The copolymer adsorbents useful in the practice of the process of the present invention may be variously treated to produce either a cationic ion exchange resin or an anionic ion exchange resin. For example sulfonation will produce a cation ion exchange resin. By way of contrast chloroalkylation of the copolymer followed by amination will yield an anionic ion exchange resin.

The conditions of emulsion polymerization to produce the adsorbents used in the process of the instant invention, particularly the choice of solvents and precipitating agents can be so chosen as to produce a copolymer that has a fairly high specific surface area along with a useful microporosity. When converted to a resin such high surface coupled with microporosity leads to a greater accessibility of the adsorption sites thereby providing a superior adsorbent. Materials with high specific surface areas tend to have surface work functions that provide a driving force for adsorption. Generally the chemical constitution of the surface will affect the types of molecules preferentially adsorbed thereon. This phenomenon then provides the basis for an adsorption purification of suitable substrates. The aforementioned copolymers are suitable for the purification of organic phases.

The instant invention comprises a process wherein an aqueous brine contaminated with an amine or amine salt and containing from about 5 to 40 weight percent of dissolved salts, i.e. a brine having a high ionic strength, is contacted with a resin having a suitable microporosity. The amine(s), amine salt(s),or mixtures thereof being a contaminant is(are) adsorbed onto the resin and the resulting purified brine may now be: 1) electrolyzed to generate chlorine or other halogens without the hazardous generation of nitrogen tri-halide compounds, 2) diluted and safely disposed, or 3) recycled as process water or brine with or without any salt make-up that may be necessary. The amine or amine salt is desorbed by washing the adsorbed resin with pure water or a brine having a significantly lower salt content, or mixtures thereof, i.e. a low ionic strength, than that from which the amine or amine salt was adsorbed on to the resin. The process of washing the resin desorbs the adsorbed amine or amine salt and regenerates the resin which may be re-used to adsorb additional amine or amine salt(s). Consequently one embodiment of the process is a cyclic process wherein amine, amine salt, or mixtures thereof is adsorbed and desorbed by a water wash which also regenerates the resin for further adsorption desorption cycles.

A preferred application involves the purification of brine solutions removing the amine or amine salts dissolved therein resulting from the interfacial condensation polymerization of bisphenol and phosgene derivatives to make polycarbonates. Referring to Figure 1, which is a flow sheet, A is an interfacial polymerization reactor for the interfacial polymerization to manufacture polycarbonates, B is a centrifuge, decanter or other device for the separation of two immiscible liquid phases, C is an optional stripping column, replaceable by H, D is a storage tank for the aqueous brine stream, E is a filter, F is a heat exchanger, G is the resin adsorption bed for adsorbing nitrogen compounds thereon, H is a stripping column which may replace the stripping column C, I is a storage tank for the purified aqueous brine solution, J is a storage tank for storing the wash solvent containing the recovered nitrogen compounds, K is a heat exchanger The process streams are as follows: 11 is an aqueous phase feed stock to the reactor, A; 12 is an organic phase feed stock to the reactor, A; 13 is the product mixture from the reactor, A, fed to the centrifuge, B; 14 is the product polymer, 15 is a brine product from the centrifuge, B, fed to a stripping column, C; 16 is a brine product fed to storage, D, from air stripping column, C; 17 is the brine fed from storage D through filter E; 18 is the brine product from the filter E to a heat exchanger, F; 19 the brine product from the heat exchanger F to the adsorption resin bed, G; 20 is the purified brine product from the adsorption resin bed G to a stripper column H if not already included; 21 is a purified brine product from the stripper column H to storage I; 22 is purified brine from storage I to various methods of salt recovery or disposal; 23 is water fed to a heat exchanger K; 24 is desorption wash water from heat exchanger K to the adsorption resin bed G; 25 is an acid solution for pH control; 26 is a dilute caustic stream for the removal of phenolic type compounds; 27 is a solution of recovered nitrogen compounds; 28 is a solution of recovered nitrogen compounds; 29 is a solution containing contaminants washed from the adsorption resin by caustic stream 26; 30, is a caustic solution to adjust the pH of the brine product feed, 19; and 31 is an acid solution to adjust the pH of the brine product feed, 19.

While Figure 1 demonstrates a possible embodiment of the present invention, other process schemes may be employed that serve the same purpose or function. Thus it is contemplated that various combinations and permutations of process functions effective for adsorbing organic compounds soluble in brines onto a polymeric resin comprise the present invention. For example, instead of a single adsorption reactor, as shown in Figure 1, two or more adsorption reactors could be used in parallel with appropriate switching valves such that while one reactor was being operated in an adsorption mode, one or more of the other reactors was being operated in a desorption and/or regeneration mode. This process configuration would have the advantage of permitting continuous operation without any need to interrupt the flow of organic compound containing brine to the resin containing adsorption column. The choice of which flow scheme would be employed would depend on large part whether or not the interfacial polymerization or other source process generating the organic containing brine was being operated in a batch or continuous mode. In the case of a batch mode, a single adsorption reactor would tend to be preferred from the standpoint of process engineering and economic considerations. In contrast, continuous processes would tend to require a parallel adsorption train that would permit continuous removal of the organic contaminants from the brine. Having disclosed and enabled the process, many variations and permutations that are equivalent in function are possible, as can be determined by a person having ordinary skill in the art.

Depending on what type of polymerization catalysts are employed in the interfacial condensation polymerization of bisphenols and phosgene derivatives to make polycarbonates, the process of the instant invention will have one of two preferred embodiments. The phase transfer catalysts typically employed in the interfacial process are amine salts. Since these salts already exist in an ionizable or ionic form, their partition between organic and aqueous phases in the various process streams of the process tends to be a function of the organic solvent or the ionic strength of the aqueous solution and the solute loads in those solutions.

In contrast, when a mixed or binary catalyst system is used, one that utilizes both a quaternary organic ammonium salt as a phase transfer catalyst and an unprotonated tri-amine as a co-catalyst, it becomes more important to consider the solubility of the tri-amine component as a function of the hydrogen ion concentration, or pH. At pH values greater than 7, tri-amines will tend to exist in aqueous solution as the solvated free amine. At pH values ranging from 7 to 14, the highly lipophilic character of organic tri-amines provides a driving force for adsorption onto the adsorbent resin. Thus the wash water utilized for desorption for the recovery of mixed catalysts adsorbed onto the adsorbent resin should have a pH below about 7, i.e. in the acid range. The acid conditions of the wash water solution protonate the adsorbed tri-amine converting it to an ammonium salt and thereby rendering it more soluble in the aqueous phase.

Desorption is facilitated by increasing the temperature of the desorbing solvent. In the case of water as the desorbing solvent recovery of the quaternary ammonium salt and any adsorbed triamine is increased by increasing the solvent temperature from about 20 °C to about 90 °C.

There are thus two modes of adsorption desorption to be utilized by the process of the present invention depending on the type of catalyst system being used in the interfacial polymerization process. The first is pH independent, while the second utilizes an acidic aqueous solution to wash or desorb the adsorbed co-catalyst and phase transfer catalysts from the adsorbent resin.

### Experimental

The following examples of a reduction to practice are illustrative of the present invention, demonstrating only specific embodiments or utility. The examples by their choice and presentation do not construe limitations to the hereinafter appended claims.

### General Procedure:

Two columns of internal dimensions 25 cm in length and 2.7 cm diameter, possessing a volume of 143.13 cc, were charged with 100g of a non-ion-exchangeable adsorbent polymeric resin copolymer. The two columns differed from each other in that one was jacketed for temperature controlled experiments and the other was not. The columns were partially filled with water and the resin introduced from the top of the column. As the column was filled, water was released from the bottom to prevent overflowing. After filling the column with wet resin, each end of the column was tightly fitted to restrict expansion of the packing material. A peristaltic pump was then used to pump one of two types of brine through the column, either a synthetic brine which was an aqueous solution of NaCl containing 20 weight percent of reaction brine from the aqueous phase of a commercial interfacial polymerization. In addition to approximately 20 weight percent NaCl, the reaction brine also contained low levels of phenols,, methylene chloride, and carbonate salts. Each brine was pumped through the resin containing column at a rate of 20 ml/min. which is a space velocity of 8.4 column or bed volumes per hour. The two individual brines were doped with various amounts of phase transfer catalysts, tertiary amines, and methylene chloride. If the jacketed column was used, the temperature of the adsorption experiment could be varied. The concentration of the brine was also varied. The column eluent was monitored to determine the efficiency and completeness of adsorption. Breakthrough was defined as when 1 wppm of phase transfer catalyst was detected in the eluent. Regeneration of the column was accomplished by stopping the flow of brine in one direction and washing with de-ionized water in the opposite direction, i.e. if adsorption was downflow, desorption was upflow and vice-versa. Elution of the catalyst was monitored by frequent sampling. Variation of the water wash flow rates from 2 to 4 ml/min. produced identical elution curves based on water volumes. The higher flow rates thus predominate in the following examples.

### Example 1.1

The column was charged with 97 g of Rohm and Haas XAD-4, a non-ion-exchangeable adsorbent polymeric resin copolymer, the synthetic brine was doped with 560 wppm methyltributylammonium chloride (MTBA). Approximately 6 kg of doped synthetic brine solution was passed through the column at 24 °C before breakthrough was observed.

### Example 1.2

Example 1.1 was repeated using a synthetic brine doped with 280 wppm MTBA. Breakthrough was observed after 12 kg of brine had passed through the column.

### Example 1.3

Example 1.1 was repeated using a synthetic brine doped with 112 wppm MTBA. Breakthrough was observed after 28 kg of doped brine had passed through the column.

### Example 2.1

Example 1.1 was repeated.

### Example 2.2

Example 2.1 was repeated except that the salt concentration of the brine was reduced to 13.5 weight percent. Breakthrough was observed after 6 kg of the brine had passed through the column.

### Example 3.1

The column was charged with 97 g of XAD-4 and commercial reaction brine was doped with 560 wppm MTBA. Approximately 8 kg of doped brine solution was passed through the column at 20 °C before breakthrough was observed.

### Example 3.2

After regeneration of the column used in Example 3.1, the column was eluted with a catalyst free commercial reaction brine containing 1000 wppm methylene chloride (1 g methylene chloride/ 1000g brine). After 16 kg of the brine had passed through the column breakthrough was observed. The adsorption of the methylene chloride caused the column to swell. The commercial reaction brine containing an additional 560 wppm MTBA was passed through the column and breakthrough was observed at 8 kg of brine.

### Example 4.1

The column was charged with 97 g of XAD-4 and commercial reaction brine was doped with 560 wppm MTBA. Approximately 8 kg of doped brine solution was passed through the column at 20 °C before breakthrough was observed.

### Example 4.2

Following regeneration of the column used in example 4.1, the identical brine was used, except that an additional 1000 wppm of triethylamine was added. The pH of this brine solution was about 10.5. After 3.4 kg of this doubly doped brine had passed through the column, breakthrough of the MTBA was observed. Both MTBA and the triethylamine adsorbed onto the column.

### Example 5.1

The column was charged wit 97 g of XAD-4 and the commercial reaction brine was doped with 560 wppm MTBA. Approximately 8 kg of doped brine solution was passed through the column at 20 °C before breakthrough was observed. Additional brine was passed through the column and the level of MTBA in the eluent was monitored.

**Table I**

| **Eluent Brine MTBA Levels After Breakthrough in Example 5.1** | |
|---|---|
| **Total Weight of Brine kg** | **Eluent MTBA Level wppm** |
| 8 | 1.16 |
| 9 | 3.02 |
| 10 | 11.7 |
| 11 | 13.9 |
| 12 | 79.1 |
| 13 | 143 |
| 14 | 163 |
| 15 | 236 |
| 16 | 395 |
| 17 | 427 |
| 18 | 476 |
| 19 | 459 |
| 20 | 464 |

### Example 6.1

The column was charged with 97 g of Rohm and Haas XAD-7, a non-ion-exchangeable adsorbent polymeric resin copolymer. The synthetic brine had salt concentration of 17.6 weight percent NaCl and was doped with 480 wppm MTBA. Approximately 2.8 kg of doped brine solution was passed through the column at 24 °C before breakthrough was observed.

### Example 7.1

Recovery of the MTBA from the XAD-4 resin used in Examples 1-5 was nearly identical in all cases since the same amount of MTBA was deposited on the resin despite different conditions. Pure water was passed through the resin containing column in a direction counter current to the direction of flow during adsorption at a flow of 4 ml/min. which is a space velocity of 1.58 ml/cc/hr. No significant levels of MTBA were observed in the first 70 g of water exiting the column during the wash. As the wash water exits the column, the level of MTBA rises quickly to a maximum value of approximately 30,000 wppm when the wash is accomplished at 20 °C. Use of 1000 g of wash water (1,000 cc) allows for recovery of approximately 90% of the MTBA adsorbed onto the column. The level of MTBA in the wash water after 1000 g of wash water had passed through the column was approximately 1000 wppm. Repeating the water wash at 80 °C, the peak level of MTBA observed in the wash water exiting the column is 46,000 wppm. Again, the level of MTBA in the wash water after 1000 g of wash water had passed through the column was approximately 1000 wppm.

### Example 8.1

The desorption procedure of Example 7.1 was followed for the nearly saturated resin as prepared in Example 5.1. Peak MTBA concentrations in the wash water varied as a function of wash water temperature. At 20 °C the peak MTBA concentration was 50,000 wppm and at 80 °C the peak MTBA concentration was 141,000 wppm.

### Example 9.1

The desorption procedure of Example 7.1 was done using the resin from Example 3. The MTBA desorbed in a fashion similar to that observed in example 7.1 with the exception that the resin shrank as methylene chloride also desorbed.

### Example 10.1

Recovery of MTBA from the sorbed resin of Example 4.2, which contained added triethylamine, using pure wash water appeared to proceed in the same manner as a normal desorption. However, a subsequent adsorption using a brine containing 560 wppm MTBA failed to treat the expected 8 kg of brine. Pure water washing apparently failed to remove all of the adsorbed triethylamine. Washing with a slightly acidic water solution, pH below about 5.5, did remove the remaining adsorbed triethylamine. At pH's above about 9.5, the free triethylamine does adsorb well onto the resin.

### Example 11.1

The resin used in Example 6.1 was water washed with pure water at a flow rate of 2 ml/min. which is a space velocity of 0.79 ml/cc/hr. Peak concentration observed for the MTBA was 11,000 wppm. Essentially all of the adsorbed MTBA was recovered with a 200 cc water wash.

### Summary of Experimental Results

Example 1 demonstrates dependence of breakthrough on the concentration of the quaternary ammonium salt, i.e. an amine salt, present in the brine, suggesting a fixed adsorption capacity for the resin. Example 2 demonstrates that adsorption of the quaternary ammonium salt onto the resin is independent of salt concentration in the brine. Example 3 shows that while other organics such as methylene chloride may adsorb onto the column, the adsorption of the quaternary ammonium salt is not inhibited thereby. Example 4 shows that the addition of other nitrogenous compounds to the brine proportionally reduces the ability of the resin to adsorb the quaternary ammonium salt. Example 5 demonstrates that the resin still retains adsorptive capacity even after breakthrough. Example 6 demonstrates adsorption using an alternative resin. Example 7 demonstrates desorption. Example 8 demonstrates an increasing efficiency of desorption with an increasing temperature of the wash water. Example 9 demonstrates that resin swollen by the adsorption of an organic compound such as methylene chloride does not interfere with desorption. Example 10 demonstrates recovery of a mixture of amine salts and tertiary amines is facilitated by the use of an acidic water wash desorption. Example 11 demonstrates the desorption using an alternative resin.

## Claims

1. A process for the adsorption of one or more amines and/or amine salt phase transfer catalysts from an aqueous solution having a high ionic strength comprising:
(a) selecting an aqueous solution comprising:
(i) a high ionic strength equivalent to a sodium chloride level present in said solution varying from 5 weight percent to 40 weight percent; and
(ii) said amines and/or amine salts;
(b) contacting said solution with a non-ion exchangeable adsorbent polymeric resin copolymer:
whereby said solution is depleted in the concentration of amines and/or amine salts by the adsorption of said amines and amine salts onto said non-ion-exchangeable adsorbent polymeric resin copolymer.

2. A process for the desorption of one or more amines and/or amine salt phase transfer catalysts adsorbed onto a non-ion-exchangeable adsorbent polymeric resin copolymer comprising:
(a) selecting a non-ion-exchangeable adsorbent polymeric resin copolymer having adsorbed thereon one or more of said amines and/or amine salts; and
(b) desorbing said adsorbed amines and/or amine salts by washing said non-ion-exchangeable adsorbent polymeric resin copolymer with an effective amount of a solvent effective to remove said amines and/or amine salts from said non-ion-exchangeable adsorbent polymeric resin copolymer,
wherein said amines and/or amine salts are dissolved in said solvent.

3. A process for adsorption and desorption comprising:
(a) adsorption of one or more amine and/or amine salt phase transfer catalyst salts from an aqueous solution having a high ionic strength comprising:
(i) selecting an aqueous solution comprising:
(1) a high ionic strength equivalent to a sodium chloride level present in said solution varying from 5 weight percent to 40 weight percent; and
(2) said amine or amine salts,
(ii) contacting said solution with a non-ion-exchangeable adsorbent polymeric resin copolymer,
whereby said solution is depleted in the concentration of said amines and/or amine salts by the adsorption of said amine compounds onto said non-ion-exchangeable adsorbent polymeric resin copolymer; and
(b) desorption of said amines and/or amine salts, said amine and/or amine salts adsorbed onto a non-ion-exchangeable adsorbent polymeric resin copolymer comprising:
(i) selecting an adsorbent polymeric resin copolymer having adsorbed thereon one or more amines and/or amine salts; and
(ii) desorbing said adsorbed amines and/or amine salts by washing said adsorbent polymeric resin copolymer with an effective amount of a solvent effective to remove said nitrogen compounds from said non-ion-exchangeable adsorbent polymeric resin copolymer,
whereby said amines and/or amine salts are dissolved in said solvent.

4. The process of claim 1, claim 2 or claim 3 wherein said amine and amine salts are selected from the group consisting of tertiary and quaternary ammonium salts.

## Patentansprüche

1. Ein Verfahren zur Adsorption von einem oder mehreren Amin- und/oder Aminsalz-Phasentransferkatalysatoren aus einer wäßrigen Lösung mit einer hohen Ionenstärke, aufweisend:
a) Auswahl einer wäßrigen Lösung, aufweisend:
i) eine hohe Ionenstärke, äquivalent zu einem in Lösung vorhandenem Natriumchloridgehalt, der von 5 Gewichtsprozent bis 40 Gewichtsprozent variiert, und
ii) Amine und/oder Aminsalze
b) Berühren der Lösung mit einem nicht-ionentauschenden, adsorbierenden, polymeren Harzcopolymer,
wobei die Konzentration an Aminen und/oder Aminsalzen durch Adsorption der Amine und Aminsalze auf dem nicht-ionentauschenden, adsorbierenden Harzcopolymer verringert wird.

2. Ein Verfahren zur Desorption von einem oder mehreren, auf einem nicht-ionentauschenden, adsorbierenden, polymeren Harzcopolymer adsorbierten Amin- und/oder Aminsalz-Phasentransferkatalysatoren, aufweisend:
a) Auswahl eines nicht-ionentauschenden, adsorbierenden, polymeren Harzcopolymers, welches darauf ein oder mehrere der Amine und/oder Aminsalze adsorbiert hat; und
b) Desorption der Amine und/oder Aminsalze durch Waschen des nichtionentauschenden, adsorbierenden, polymeren Harzcopolymers mit einer effektiven Menge eines Lösungsmittels, das effektiv ist, um die Amine und/oder Aminsalze vom nicht-ionentauschenden, adsorbierenden, polymeren Harzcopolymer zu entfernen;
wobei die Amine und/oder Aminsalze im Lösungsmittel gelöst werden.

3. Ein Verfahren zur Adsorption und Desorption, aufweisend:
a) Adsorption von einem oder mehreren Amin- und/oder Aminsalz-Phasentransferkatalysatorsalzen aus einer wäßrigen Lösung mit einer hohen Ionenstärke, aufweisend:
i) Auswahl einer wäßrigen Lösung, aufweisend:
(1) eine hohe Ionenstärke, äquivalent zu einem in Lösung vorhandenem Natriumchloridgehalt, der von 5 Gewichtsprozent bis 40 Gewichtsprozent variiert, und
(2) Amine und/oder Aminsalze
ii) Berühren der Lösung mit einem nicht-ionentauschenden, adsorbierenden, polymeren Harzcopolymer,
wobei die Lösung in ihrer Konzentration an Aminen und/oder Aminsalzen verringert wird durch die Adsorption der Aminverbindungen auf dem nicht-ionentauschenden, adsorbierenden, polymeren Harzcopolymer; und
b) Desorption der Amine und/oder Aminsalze, wobei die Amine und/oder Aminsalze auf dem nicht-ionentauschenden, adsorbierenden, polymeren Harzcopolymer adsorbiert sind, aufweisend:
i) Auswahl eines nicht-ionentauschenden, adsorbierenden, polymeren Harzcopolymers, welches darauf ein oder mehrere Amine und/oder Aminsalze adsorbiert hat; und
b) Desorption der adsorbierten Amine und/oder Aminsalze durch Waschen des nicht-ionentauschenden, adsorbierenden, polymeren Harzcopolymers mit einer effektiven Menge eines Lösungsmittels, das effektiv ist, um die Stickstoffverbindungen vom nicht-ionentauschenden, adsorbierenden, polymeren Harzcopolymer zu entfernen;
wobei die Amine und/oder Aminsalze im Lösungsmittel gelöst werden.

4. Das Verfahren gemäß Anspruch 1, Anspruch 2 oder Anspruch 3, wobei die Amine und Aminsalze aus der aus tertiären und quaternären Ammoniumsalzen bestehenden Gruppe ausgewählt werden.

## Revendications

1. Procédé d'adsorption d'un ou plusieurs catalyseurs de transfert de phase, constitués d'amines et/ou de sels d'amine, à partir d'une solution aqueuse ayant une force ionique élevée, qui comprend les étapes consistant à :
(a) choisir une solution aqueuse ayant (i) une force ionique élevée, correspondant à une teneur en chlorure de sodium présent dans ladite solution allant de 5 % en poids à 40 % en poids, et contenant (ii) lesdites amines et/ou lesdits sels d'amine, et
(b) mettre ladite solution en contact avec un copolymère qui est une résine polymère adsorbante, ne pouvant pas donner lieu à des échanges d'ions,
grâce à quoi ladite solution est appauvrie en amines et/ou sels d'amine par l'adsorption desdites amines et desdits sels d'amine sur ledit copolymère qui est une résine polymère adsorbante, ne pouvant pas donner lieu à des échanges d'ions.

2. Procédé pour la désorption d'un ou plusieurs catalyseurs de transfert de phase, constitués d'amines et/ou de sels d'amine, adsorbés sur un copolymère qui est une résine polymère adsorbante, ne pouvant pas donner lieu à des échanges d'ions, procédé qui comprend les étapes consistant à :
(a) choisir un copolymère qui est une résine polymère adsorbante, ne pouvant pas donner lieu à des échanges d'ions, sur lequel est adsorbé un ou plusieurs desdites amines et/ou desdits sels d'amine, et
(b) désorber lesdites amines et/ou lesdits sels d'amine adsorbés en lavant ledit copolymère qui est une résine polymère adsorbante, ne pouvant pas donner lieu à des échanges d'ions, avec une quantité efficace d'un solvant apte à éliminer lesdites amines et/ou lesdits sels d'amine dudit copolymère qui est une résine polymère adsorbante, ne pouvant pas donner lieu à des échanges d'ions,
opération lors de laquelle lesdites amines et/ou lesdits sels d'amine sont dissous dans ledit solvant.

3. Procédé d'adsorption et de désorption qui comprend :
(a) l'adsorption d'un ou plusieurs catalyseurs de transfert de phase, constitués d'amines et/ou de sels d'amine, à partir d'une solution aqueuse ayant une force ionique élevée, opération qui comprend :
(i) le choix d'une solution aqueuse ayant (1) une force ionique élevée correspondant à une teneur en chlorure de sodium présent dans ladite solution allant de 5 % en poids à 40 % en poids, et contenant (2) lesdites amines ou lesdits sels d'amine,
(ii) la mise de ladite solution en contact avec un copolymère qui est une résine polymère adsorbante, ne pouvant pas donner lieu à des échanges d'ions,
grâce à quoi ladite solution est appauvrie en lesdites amines et/ou lesdits sels d'amine par l'adsorption desdits composés aminés sur ledit copolymère qui est une résine polymère adsorbante, ne pouvant pas donner lieu à des échanges d'ions, et
(b) la désorption desdites amines et/ou desdits sels d'amine adsorbés sur un copolymère qui est une résine polymère adsorbante, ne pouvant pas donner lieu à des échanges d'ions, opération qui comprend :
(i) le choix d'un copolymère qui est une résine polymère adsorbante, sur lequel est adsorbé un ou plusieurs amines et/ou sels d'amine, et
(ii) la désorption desdites amines et/ou sels d'amine adsorbés par un lavage dudit copolymère qui est une résine polymère adsorbante par une quantité efficace d'un solvant apte à éliminer lesdits composés azotés
dudit copolymère qui est une résine polymère adsorbante, ne pouvant pas donner lieu à des échanges d'ions,
opération lors de laquelle lesdites amines et/ou lesdits sels d'amine sont dissous dans ledit solvant.

4. Procédé selon l'une quelconque des revendications 1 à 3, dans lequel lesdites amines et lesdits sels d'amine sont choisis parmi les sels d'amine tertiaire et les sels d'ammonium quaternaires.
